# EUROPEAN PATENT APPLICATION

(11) **EP 3 466 352 A1**
(43) Date of publication of application: **10.04.2019**
(21) Application number: 16903110.1
(22) Date of filing: 25.05.2016
(51) Int. Cl.: A61B 17/28, A61B 17/32, A61B 18/14

(54) **GRIPPING TREATMENT INSTRUMENT**

(71) Applicant: Olympus Corporation, Hachioji-shi, Tokyo 192-8507 (JP)
(72) Inventor: HIRAI, Yuji, Hachioji-shi Tokyo 192-8507 (JP); NAGANO, Shunichi, Hachioji-shi Tokyo 192-8507 (JP)
(74) Representative: Gunzelmann, Rainer
(86) International application number: PCT/JP2016/065455
(87) International publication number: WO 2017/203628

(57) **Abstract**

A grasping treatment instrument includes a sheath extending along a longitudinal axis, a first grasping piece provided at a distal end portion of the sheath, a second grasping piece moving rotationally relative to the sheath, thereby opening and closing relative to the first grasping piece to grasp a living tissue between the second grasping piece and the first grasping piece, a protrusion provided on the second grasping piece and protruding in a direction intersecting with the longitudinal axis and a direction in which the second grasping piece opens and closes, and a recess that engages with the protrusion at the distal end portion of the sheath and has a recessed shape that opens in a direction intersecting with the direction to which the protrusion protrudes.

## Description

### Technical Field

The present invention relates to a grasping treatment instrument that grasps a treatment target between a pair of grasping pieces.

### Background Art

US 2010/0057117 A1 discloses a grasping treatment instrument that grasps a treatment target between a pair of grasping pieces. In such a grasping treatment instrument, at least one of a pair of grasping pieces is rotatably attached to a distal end portion of a sheath. Then, at least one of the grasping pieces moves rotatably relative to the sheath, whereby one of the pair of grasping pieces opens or closes relative to the other grasping piece.

### Summary of Invention

In a grasping treatment instrument as disclosed in U.S. Patent Application Publication No. 2010-057117, a hole is provided in each of a sheath and a grasping piece rotatably attached to the sheath. Then, by inserting a pin into these holes, the grasping piece is rotatably attached to the sheath. When a grasping piece is attached to a sheath using a pin as described above, the work of attaching the grasping piece to the sheath becomes troublesome.

The present invention has been made to solve the above problem, and an object of the present invention is to provide a grasping treatment instrument allowing the grasping pieces to be easily attached to the sheath in a configuration where at least one of a pair of grasping pieces moves rotationally relative to a sheath.

To solve the above mentioned problems, according to one aspect of the invention, a grasping treatment instrument including: a sheath having a longitudinal axis and extending along the longitudinal axis, a first grasping piece provided at a distal end portion of the sheath, a second grasping piece attached to the distal end portion of the sheath so as to be rotationally movable relative to the sheath, the second grasping piece moving rotationally relative to the sheath, thereby opening and closing relative to the first grasping piece to grasp a living tissue between the second grasping piece and the first grasping piece, a protrusion provided on the second grasping piece and protruding in a direction intersecting with the longitudinal axis and intersecting with a direction in which the second grasping piece opens and closes relative to the first grasping piece, and a recess that engages with the protrusion at the distal end portion of the sheath and has a recessed shape that opens in a direction intersecting with the direction to which the protrusion protrudes.

### Brief Description of Drawings

FIG. 1 is a schematic diagram showing a grasping treatment instrument according to a first embodiment.
FIG. 2 is a perspective view showing a second grasping piece, a sheath, and a movable member according to the first embodiment.
FIG. 3 is a perspective view showing the configuration of the second grasping piece according to the first embodiment.
FIG. 4 is a perspective view showing the configuration of the sheath and the movable member according to the first embodiment.
FIG. 5 is a perspective view showing a pressing member according to the first embodiment.
FIG. 6 is a schematic diagram showing the sheath, an end effector, the movable member, and the pressing member according to the first embodiment.
FIG. 7 is a cross-sectional view taken along line A-A of FIG. 6.
FIG. 8 is a schematic diagram showing the configuration of a sheath, a second grasping piece, and a movable member according to a second embodiment.
FIG. 9 is a perspective view showing a state where the second grasping piece and the sheath according to the second embodiment are disengaged from each other.
FIG. 10 is a schematic diagram showing the configuration of a sheath, a second grasping piece, and a movable member according to a first modification of the second embodiment.
FIG. 11 is a schematic diagram showing a connecting portion (a second connecting portion) between a second grasping piece and a sheath, according to a second modification of the second embodiment, in a state where the second grasping piece is closed to some extent relative to a first grasping piece.
FIG. 12 is a schematic diagram showing the connecting portion (the second connecting portion) between the second grasping piece and the sheath, according to the second modification of the second embodiment, in a state where the second grasping piece is opened from the state shown in FIG. 11 to some extent relative to the first grasping piece.
FIG. 13 is a schematic diagram showing the connecting portion (the second connecting portion) between the second grasping piece and the sheath, according to the second modification of the second embodiment, in a state where the second grasping piece is the most open relative to the first grasping piece (a state where the second grasping piece further opened from the state shown in FIG. 12).
FIG. 14 is a schematic diagram showing a protrusion provided on a second grasping piece according to a third embodiment.
FIG. 15 is a cross-sectional view showing a connecting portion (a second connecting portion) between the second grasping piece and a sheath, according to the third embodiment, in a cross section that is substantially parallel to the protruding direction of the protrusion and passes through an inclined surface of the protrusion.
FIG. 16 is a cross-sectional view showing the second connecting portion in a state where the second grasping piece and the sheath according to the third embodiment are disengaged from each other in a cross section that is substantially parallel to the protruding direction of the protrusion and passes through the inclined surface of the protrusion.
FIG. 17 is a schematic diagram showing a support member of a second grasping piece according to a reference embodiment.

### Description of Embodiments

### (First Embodiment)

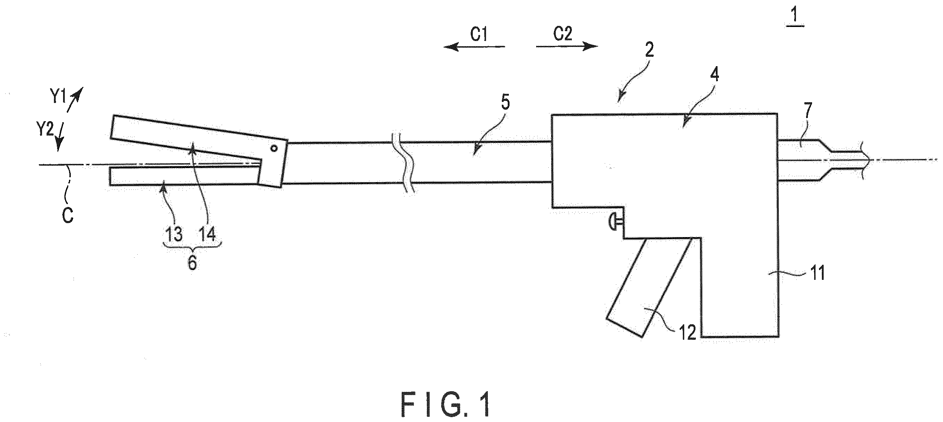

A first embodiment of the present invention will be described with reference to FIGS. 1 to 6. FIG. 1 is a diagram showing a grasping treatment instrument 1 of the present embodiment. As shown in FIG. 1, the grasping treatment instrument 1 has a longitudinal axis C. Here, in the grasping treatment instrument 1, one side in the direction along the longitudinal axis C is defined as a distal side (the arrow C1 side), and the opposite side of the distal side is defined as a proximal side (the arrow C2 side).

The grasping treatment instrument 1 includes a holdable housing 4, a sheath 5 connected to the distal side of the housing 4, and an end effector 6 provided at a distal end portion of the sheath 5. One end of a cable 7 is connected to the housing 4. The other end of the cable 7 is detachably connected to an unillustrated power supply unit. A grip (fixed handle) 11 is provided to the housing 4, and a handle (movable handle) 12 is rotatably attached to the housing 4. The handle 12 moves rotationally relative to the housing 4, whereby the handle 12 opens and closes relative to the grip 11.

In the present embodiment, the handle 12 is positioned on the distal side relative to the grip 11 and moves substantially in parallel with the longitudinal axis C in an opening or closing movement relative to the grip 11, but the present embodiment is not limited thereto. For example, in a certain embodiment, the handle 12 may be positioned on the proximal side relative to the grip 11. In another embodiment, the handle 12 may be positioned on the opposite side of the grip 11 relative to the longitudinal axis C, and the moving direction of the handle 12 in the opening or closing movement relative to the grip 11 may intersect with (and may be substantially perpendicular to) the longitudinal axis C.

The sheath 5 extends along the longitudinal axis C. The end effector 6 includes a first grasping piece 13 and a second grasping piece 14 that opens and closes relative to the first grasping piece 13. The handle 12 and the second grasping piece 14 are connected via a movable member (shaft) 17 that extends along the longitudinal axis C inside the sheath 5. By causing the handle 12, which is an opening/closing operation input section, to open or close relative to the grip 11, the movable member 17 moves along the longitudinal axis C relative to the sheath 5 and the housing 4, and the space between a pair of the grasping pieces 13 and 14 is opened or closed relative to each other. Here, the handle 12 opens and closes relative to the grip 11 only in a predetermined movement range. Therefore, the movable member 17 can move within a predetermined movement range along the longitudinal axis C relative to the sheath 5 and the housing 4. In the present embodiment, when the handle 12 is the most open relative to the grip 11, the movable member 17 moves to the most proximal side relative to the sheath 5 and the housing 4, and the space between the grasping pieces 13 and 14 becomes the most open. Also, when the handle 12 is the most closed relative to the grip 11, the movable member 17 moves to the most distal side relative to the sheath 5 and the housing 4, and the space between the grasping pieces 13 and 14 becomes the most closed. The space between the grasping pieces 13 and 14 is closed, whereby a living tissue such as a blood vessel is grasped as a treatment target between the grasping pieces 13 and 14. The opening/closing direction (the direction of an arrow Y1 and an arrow Y2) of the end effector 6 intersects with (and is substantially perpendicular to) the longitudinal axis C. A direction intersecting with the longitudinal axis C and intersecting with the opening/closing direction of the end effector 6 is defined as a width direction of the end effector 6 (sheath 5).

In the present embodiment, the second grasping piece 14 is attached to the distal end portion of the sheath 5 so as to move rotationally. It is sufficient that the first grasping piece 13 is provided at the distal end portion of the sheath 5 and is configured to be able to open and close relative to the second grasping piece 14. For example, in one embodiment, the first grasping piece 13 is integral with or fixed on the sheath 5. In another embodiment, the first grasping piece 13 is also attached to the distal end portion of the sheath 5 so as to move rotationally. In yet another embodiment, an unillustrated rod member is inserted through the sheath 5, and the first grasping piece 13 is formed by a projecting portion of a rod member (probe) from the sheath 5 to the distal side. Also, in one embodiment, an unillustrated rotational operation knob may be attached to the housing 4. In this case, by rotating the rotational operation knob around the longitudinal axis C relative to the housing 4, the sheath 5 and the end effector 6 rotate together with the rotational operation knob around the longitudinal axis C relative to the housing 4. With this rotation, the angular position around the longitudinal axis C of the end effector 6 is adjusted.

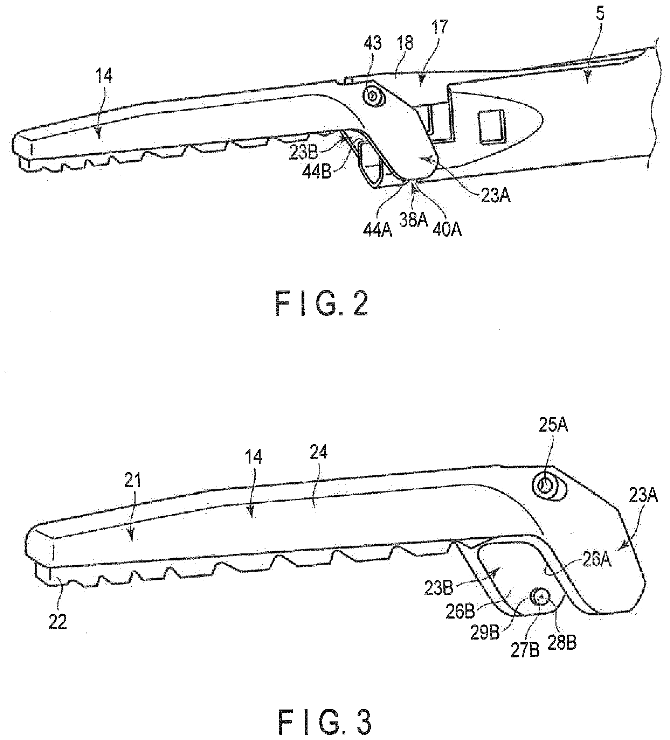

FIG. 2 is a diagram showing the sheath 5, the second grasping piece 14, and the movable member 17. FIG. 3 is a diagram showing the configuration of the second grasping piece 14. As shown in FIGS. 2 and 3, the second grasping piece 14 includes a support member 21. A clamp part 22 is attached to the support member 21. The clamp part 22 includes, for example, an unillustrated electrode formed of a conductive material. The clamp part 22 faces the first grasping piece 13. When the space between the second grasping piece 14 and the first grasping piece 13 is closed and a treatment target is grasped between the second grasping piece 14 and the first grasping piece 13, the clamp part 22 and the first grasping piece 13 are in contact with the treatment target.

The support member 21 includes a member main body 24, and two projecting pieces 23A and 23B (first projecting piece 23A and second projecting piece 23B) projecting from the proximal end of the member main body 24 toward the proximal side. Each of the projecting pieces 23A and 23B extends from the proximal end of the member main body 24 toward the side where the second grasping piece 14 is closed. The projecting pieces 23A and 23B are provided apart from each other in the width direction of the second grasping piece 14. A hole 25A penetrating the projecting piece 23A in the width direction is provided at the proximal end portion of the projecting piece 23A. A hole 25B penetrating the projecting piece 23B in the width direction is provided at the proximal end portion of the projecting piece 23B. The hole 25A and the hole 25B face each other in the width direction.

Furthermore, the projecting piece 23A includes an opposed surface 26A that faces the projecting piece 23B in the width direction. The projecting piece 23B includes an opposed surface 26B that faces the projecting piece 23A (opposed surface 26A) in the width direction. On the opposed surface 26A, a protrusion 27A protruding from the opposed surface 26A toward the opposed surface 26B (inside the second grasping piece 14 in the width direction) is provided. On the opposed surface 26B, a protrusion 27B protruding from the opposed surface 26B toward the opposed surface 26A (inside the second grasping piece 14 in the width direction) is provided. In this embodiment, the protrusions 27A and 27B protrude towards the inside of the second grasping piece 14 in the width direction of the end effector 6 (sheath 5), which is a direction intersecting with (or substantially perpendicular to) the longitudinal axis C and intersecting with (or substantially perpendicular to) the opening/closing direction of the second grasping piece 14 relative to the first grasping piece 13. The protrusion (27A, 27B) has a center axis (28A, 28B) in a cross section intersecting with (or substantially perpendicular to) the width direction, and has a circumferential surface (29A, 29B) centering on the center axis (28A, 28B). The circumferential surfaces 29A and 29B form side surfaces (outer circumferential surfaces) of the protrusions 27A and 27B. That is, each of the protrusions 27A and 27B has a circular (circular arc-shaped) curved surface in a cross section intersecting with (or substantially perpendicular to) the width direction (the direction in which the protrusions 27A and 27B protrude).

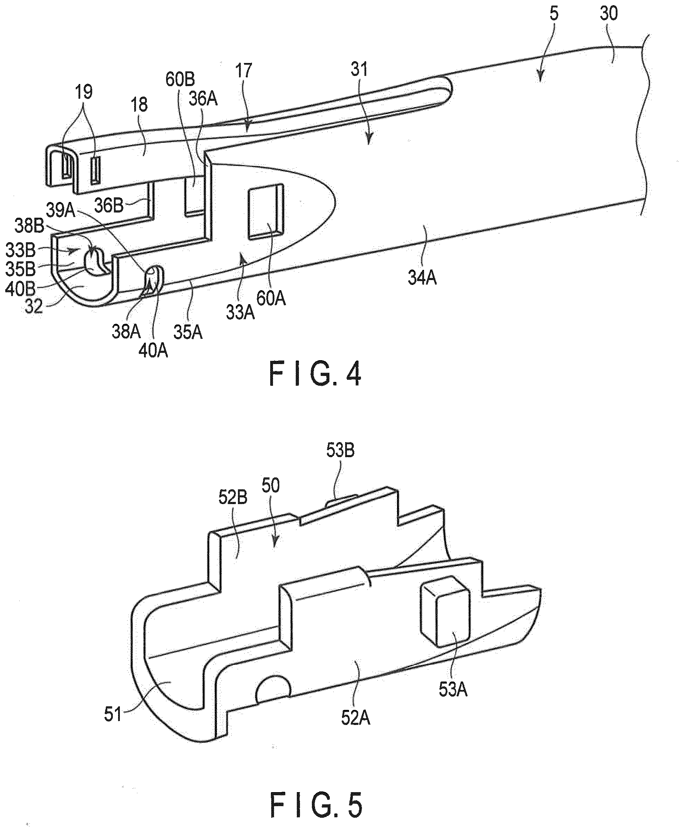

FIG. 4 is a diagram showing the configuration of the sheath 5 and the movable member 17. As shown in FIG. 4, the sheath 5 includes a tubular portion (cylindrical portion) 30 extending from the inside of the housing 4 toward the distal side, and a U-shaped portion 31 provided at the distal end portion of the sheath 5. The U-shaped portion 31 is continuous with the distal side of the cylindrical portion 30. The U-shaped portion 31 opens toward the side where the second grasping piece 14 opens relative to the first grasping piece 13 in a cross section intersecting with (or substantially perpendicular to) the longitudinal axis C. The size of the U-shaped portion 31 at the distal end thereof in the width direction is smaller (narrower) than the size of the U-shaped portion 31 at the proximal end thereof in the width direction.

The U-shaped portion 31 includes a bottom surface portion 32 forming a U-shaped bottom and side surface portions 33A and 33B provided on both sides of the bottom surface portion 32 in the width direction. The side surface portions 33A and 33B include proximal-side side surface portions 34A, 34B and distal-side side surface portions 35A, 35B provided on the distal sides of the proximal-side side surface portions 34A and 34B. With respect to the opening/closing direction of the second grasping piece 14 (end effector 6), the length of the proximal-side side surface portions 34A, 34B is longer than the length of the distal-side side surface portions 35A, 35B. Therefore, a step 36A, 36B in the opening/closing direction of the grasping pieces 13 and 14 (end effector 6) is formed between (the distal end of) the proximal-side side surface portion 34A, 34B and (the proximal end of) the distal-side side surface portion 35A, 35B.

Inside the sheath 5, the movable member 17 extends along the longitudinal axis C. The movable member 17 is provided with an unillustrated tubular portion extending from the inside of the housing 4 toward the distal side, and a projecting portion 18 projecting from the distal end of the tubular portion (cylindrical portion) toward the distal side. The projecting portion 18 is provided at the distal end portion of the movable member 17. The projecting portion 18 extends between the side surface portion 33A and the side surface portion 33B. Furthermore, the projecting portion 18 is positioned on the opposite side of the bottom surface portion 32 relative to the longitudinal axis C in a cross section intersecting with (or substantially perpendicular to) the longitudinal axis C. A hole 19 is provided at the distal end portion of the projecting portion 18. The hole 19 penetrates the projecting portion 18 in the width direction of the second grasping piece 14 (the end effector 6).

As shown in FIG. 2, in a state where the second grasping piece 14 is attached to the sheath 5, the distal end portion of the projecting portion 18 of the movable member 17 is positioned between the projecting piece 23A and the projecting piece 23B in the width direction. The hole 19 provided at the projecting portion 18 is positioned between the hole 25A and the hole 25B in the width direction. An unillustrated connecting pin, etc., is inserted through the hole 25A, the hole 19, and the hole 25B. That is, a first connecting portion 43 is formed by the hole 25A, the hole 19, the hole 25B, and the connecting pin, etc., and the movable member 17 is connected to the second grasping piece 14 at a portion (first connecting portion 43) different from that of the protrusions 27A and 27B. As the movable member 17 moves along the longitudinal axis C relative to the sheath 5, a driving force that rotates the second grasping piece 14 relative to the sheath 5 at the first connecting portion 43 acts on the second grasping piece 14.

In the U-shaped portion 31, a hole 38A extends from the side surface portion 33A to the bottom surface portion 32. Furthermore, in the U-shaped portion 31, a hole 38B extends from the side surface portion 33B to the bottom surface portion 32. In a cross section intersecting with (or substantially perpendicular to) the width direction, the hole 38A, 38B includes a circular arc portion 39A, 39B which is a circular arc-shaped curved surface. The center of the circular arc shape in the circular arc portion 39A, 39B is positioned closer to the side where the second grasping piece 14 closes relative to the first grasping piece 13 than the circular arc portion 39A, 39B. When viewed from one side in the width direction, the holes 38A and 38B are opened toward the side where the second grasping piece 14 closes relative to the first grasping piece 13. That is, the hole 38A, 38B includes an opening 40A, 40B that opens toward the side where the second grasping piece 14 closes relative to the first grasping piece 13. By the holes 38A and 38B being formed as described above, the hole (recess) 38A, 38B has a recessed shape that opens in a direction intersecting with the direction in which the protrusions 27A and 27B protrude.

As shown in FIG. 2, in a state where the second grasping piece 14 is attached to the sheath 5, the distal end portion of the sheath 5 (U-shaped portion 31) is positioned between the projecting piece 23A and the projecting piece 23B in the width direction. The protrusion 27A provided on the projecting piece 23A engages with the hole 38A of the sheath 5, and the protrusion 27B provided on the projecting piece 23B engages with the hole 38B of the sheath 5. With this configuration, the second grasping piece 14 is connected to the sheath 5 so as to move rotationally. That is, a second connecting portion 44A is formed by the protrusion 27A and the hole 38A, and a second connecting portion 44B is formed by the protrusion 27B and the hole 38B.

When the movable member 17 moves along the longitudinal axis C relative to the sheath 5 by operation input at the handle 12, a driving force that rotationally moves the second grasping piece 14 relative to the sheath 5 acts on the second grasping piece 14 at the first connecting portion 43. Then, the second grasping piece 14 moves rotationally (rotates) relative to the sheath 5 using the second connecting portions 44A and 44B as a fulcrum, and the second grasping piece 14 opens or closes relative to the first grasping piece 13.

When the movable member 17 moves to the proximal side relative to the sheath 5 by operation input on the handle 12, a driving force that rotationally moves the second grasping piece 14 relative to the sheath 5 acts on the second grasping piece 14 at the first connecting portion 43. Then, the second grasping piece 14 moves rotationally relative to the sheath 5 using the second connecting portion 44A, 44B as a fulcrum, and the second grasping piece 14 opens relative to the first grasping piece 13. As the second grasping piece 14 opens relative to the first grasping piece 13, the angle between the second grasping piece 14 and the first grasping piece 13 increases. When the second grasping piece 14 is opened to a certain extent (to a certain angle) from a closed state relative to the first grasping piece 13, the protrusion 27A, 27B of the second grasping piece 14 moves rotationally, at the second connecting portion 44A, 44B, relative to the hole 38A, 38B in a state of contacting the circular arc portion 39A, 39B of the hole 38A, 38B. At this time, the curved surface of the protrusion 27A, 27B and the curved surface of the hole 38A, 38B (the circumferential surface 29A, 29B and the circular arc portion 39A, 39B) contact each other. Therefore, friction occurring between the circumferential surface 29A, 29B and the circular arc portion 39A, 39B at the second connecting portion 44A, 44B is reduced, and the protrusion 27A, 27B becomes easy to rotate relative to the hole 38A, 38B.

When the second grasping piece 14 is further opened from the state of being opened to a certain degree (at an angle) relative to the first grasping piece 13, the protrusion 27A, 27B moves toward the opening 40A, 40B at the second connecting portion 44A, 44B (the hole 38A, 38B). That is, between a state where the second grasping piece 14 is opened to a certain degree (at an angle) relative to the first grasping piece 13 and a state where the second grasping piece 14 is the most open relative to the first grasping piece 13, as the second grasping piece 14 opens relative to the first grasping piece 13, the protrusion 27A, 27B moves toward the opening 40A, 40B of the recess shaped hole 38A, 38B. When the movable member 17 moves to the most proximal side relative to the sheath 5, the second grasping piece 14 is in the state of being the most open relative to the first grasping piece 13. At this time, the protrusion 27A, 27B is positioned further inside the hole 38A, 38B than the opening 40A, 40B (the side that the second grasping piece 14 opens relative to the first grasping piece 13) in the hole 38A, 38B (the second connecting portion 44A, 44B).

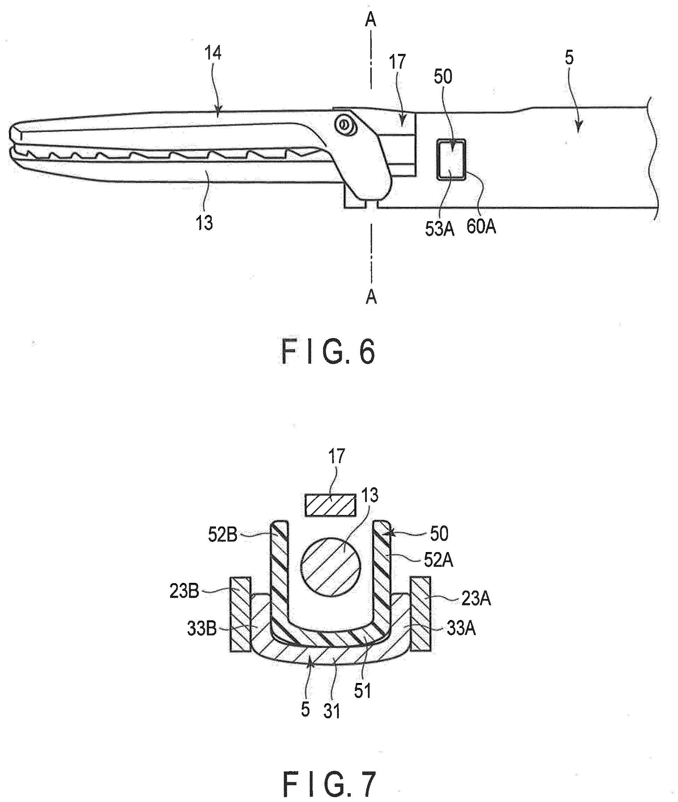

In one embodiment, the pressing member 50 is attached inside the sheath 5. In this case, a rod member is inserted through the sheath 5, and the first grasping piece 13 is formed by a projecting portion of the rod member (probe) from the sheath 5 to the distal side. By providing the pressing member 50 inside the sheath 5, contact between the rod member and the sheath 5 is prevented. FIG. 5 is a diagram showing the configuration of the pressing member 50 in the present embodiment. FIG. 6 is a diagram showing a state where a rod member (first grasping piece 13) is inserted through the sheath 5, and the pressing member 50 and the second grasping piece 14 are attached to the sheath 5. As shown in FIG. 6, the sheath 5 is provided with a hole 60A, 60B penetrating each of the proximal-side side surface portions 34A and 34B in the width direction.

As shown in FIG. 5, the pressing member 50 has a U-shape in a cross section intersecting with (or substantially perpendicular to) the longitudinal axis C, and extends along the longitudinal axis C. The pressing member 50 includes a bottom surface portion 51 forming a U-shaped bottom portion in a cross section intersecting with the longitudinal axis C, and side surface portions 52A and 52B provided on both sides of the bottom surface portion 51 in the width direction and forming U-shaped side walls. The length of the pressing member 50 in the width direction (the length from the outer surface of the side surface portion 52A to the outer surface of the side surface portion 52B) is shorter than the length of the U-shaped portion 31 of the sheath 5 in the width direction (the length from the inner surface of the side surface portion 33A to the inner surface of the side surface portion 33B). A protrusion 53A, protruding toward the outside of the side surface portion 52A in the width direction, is provided on the side surface portion 52A. Furthermore, on the side surface portion 52B, a protrusion 53B, protruding toward the outside of the side surface portion 52B in the width direction, is provided. As shown in FIGS. 5 and 6, in a state where the pressing member 50 is attached to the sheath 5, the protrusion 53A engages with a hole 60A and the protrusion 53B engages with a hole 60B. With this configuration, the movement of the pressing member 50 relative to the sheath 5 is restricted. That is, by the engagement of the protrusion 53A, 53B with the hole 60A, 60B, the pressing member 50 is effectively fixed on the sheath 5.

FIG. 7 is a diagram showing a cross-sectional surface taken along the line A-A in FIG. 6. As shown in FIG. 7, in a state where the second grasping piece 14 is closed relative to the first grasping piece 13, a side surface portion 33A, 33B (distal-side side surface portion 35A, 35B) is positioned on both sides of the movable member 17 and the probe (rod member) in the width direction. On the outside of the side surface portion 33A in the width direction, the projecting piece 23A of the second grasping piece 14 is positioned. Furthermore, on the outside of the side surface portion 33B in the width direction, the projecting piece 23B of the second grasping piece 14 is positioned. A pressing member 50 is placed inside the U-shaped portion 31 of the sheath 5. Therefore, the corresponding side surface portion from 52A and 52B of the pressing member 50 is placed inside the side surface portions 33A and 33B of the sheath 5 in the width direction. Therefore, when a treatment target such as a living tissue is treated, the side surface portions 52A and 52B prevent a living tissue, etc. from flowing into the inside of the sheath 5 from the outside in the width direction. A living tissue, etc. is prevented from flowing into the inside of the sheath 5, so that occurrence of tissue clogging by the living tissue, etc. inside the sheath 5 is effectively prevented.

Next, the operation and effects of the present embodiment will be described. When treatment is performed using the grasping treatment instrument 1 of the present embodiment, the operator holds the housing 4 and places a treatment target such as a living tissue between the grasping pieces 13 and 14. Then, by causing the handle 12 to close relative to the grip 11, the space between the grasping pieces 13 and 14 is closed. With this, the treatment target is grasped between the grasping pieces 13 and 14.

In the manufacture of the grasping treatment instrument 1, the second grasping piece 14 is attached to each of the sheath 5 and the movable member 17 in a state where the movable member 17 is placed inside the sheath 5. In the present embodiment, first, the second grasping piece 14 is attached to the sheath 5. When the second grasping piece 14 is attached to the sheath 5, the protrusion 27A, 27B of the second grasping piece 14 is inserted into the hole 38A, 38B of the sheath 5 from the opening 40A, 40B toward the opposite side of the direction in which the hole 38A, 38B opens. Then, the protrusion 27A, 27B inserted from the opening 40A, 40B comes into contact with the circular arc portion 39A, 39B. The protrusion 27A, 27B is brought into contact with the circular arc portion 39A, 39B, whereby the second connecting portion 44A, 44B is formed. Then, the second grasping piece 14 is connected to the sheath 5 so as to move rotationally.

Next, in a state where the second grasping piece 14 is connected to the sheath 5 so as to move rotationally, the second grasping piece 14 is attached to the movable member 17. When the second grasping piece 14 is attached to the movable member 17, the second grasping piece 14 is caused to move rotationally relative to the sheath 5 using the second connecting portion 44A, 44B as a fulcrum so that the grasping piece 14 is placed in a state where the hole 19 provided in the projecting portion 18 is positioned to between the holes 25A and 25B. Then, in a state where the hole 19 provided in the projecting portion 18 is positioned between the holes 25A and 25B, a connecting pin, etc. is inserted through the hole 25A, the hole 19 and the hole 25B. By inserting a connecting pin, etc. through the hole 25 A, the hole 19 and the hole 25 B, the second grasping piece 14 is connected to the movable member 17. Then, the first connecting portion 43 is formed by the holes 25A, 25B and the hole 19.

In the present embodiment, the holes 38A and 38B provided in the sheath 5 are opened toward the direction intersecting with the protruding direction of the protrusion 27A, 27B provided on the second grasping piece 14. Therefore, by inserting the protrusion 27A, 27B into the hole 38A, 38B from the opening 40A, 40B and moving the protrusion 27A, 27B toward the opposite side of the opening direction in the hole 38A, 38B, the second grasping piece 14 and the sheath 5 can be connected in the connecting portion (the second connecting portion 44A and 44B). Therefore, the step of inserting a connecting pin, etc. into members to be connected (namely, the sheath 5 and the second grasping piece 14) is rendered unnecessary, and the second grasping piece 14 can be easily attached to the sheath 5.

Furthermore, in the sheath 5, the hole 38A, 38B is provided on the opposite side of the projecting portion 18 and the hole 19 of the movable member 17 relative to the longitudinal axis C. Therefore, the second connecting portion 44A, 44B formed by the hole 38A, 38B and the protrusion 27A, 27B is positioned on the opposite side of the first connecting portion 43 formed by the hole 19, the hole 25A, 25B, and the connecting pin, etc., relative to the longitudinal axis C. The second connecting portion 44A, 44B is positioned on the opposite side of the first connecting portion 43 relative to the longitudinal axis C, whereby a distance between the second connecting portion 44A, 44B and the first connecting portion 43 is secured in the sheath 5. Here, when the second grasping piece 14 opens and closes relative to the first grasping piece 13, a driving force of opening and closing the second grasping piece 14 is transmitted from the movable member 17 to the second grasping piece 14 at the first connecting portion 43, and the second grasping piece 14 moves rotationally using the second connecting portion 44A, 44B as a fulcrum. That is, when the second grasping piece 14 opens and closes relative to the first grasping piece 13, the second connecting portions 44A and 44B serves as a fulcrum of the rotation of the second grasping piece 14, and the first connecting portion 43 serves as a force point of the rotation of the second grasping piece 14. Therefore, by securing, in the sheath 5, a distance between the second connecting portions 44A, 44B and the first connecting portion 43, a distance between the fulcrum and the force point of the rotation of the second grasping piece 14 is secured. By securing the distance between the fulcrum and the force point of the rotation of the second grasping piece 14, the force applied to the force point when causing the second grasping piece 14 to move rotationally relative to the sheath 5 decreases. Therefore, it is possible to cause the second grasping piece 14 to move rotationally relative to the sheath 5 and to open and close relative to the first grasping piece 13 with a relatively small force (even if the operational force applied to the handle 12 is small).

### (Second Embodiment)

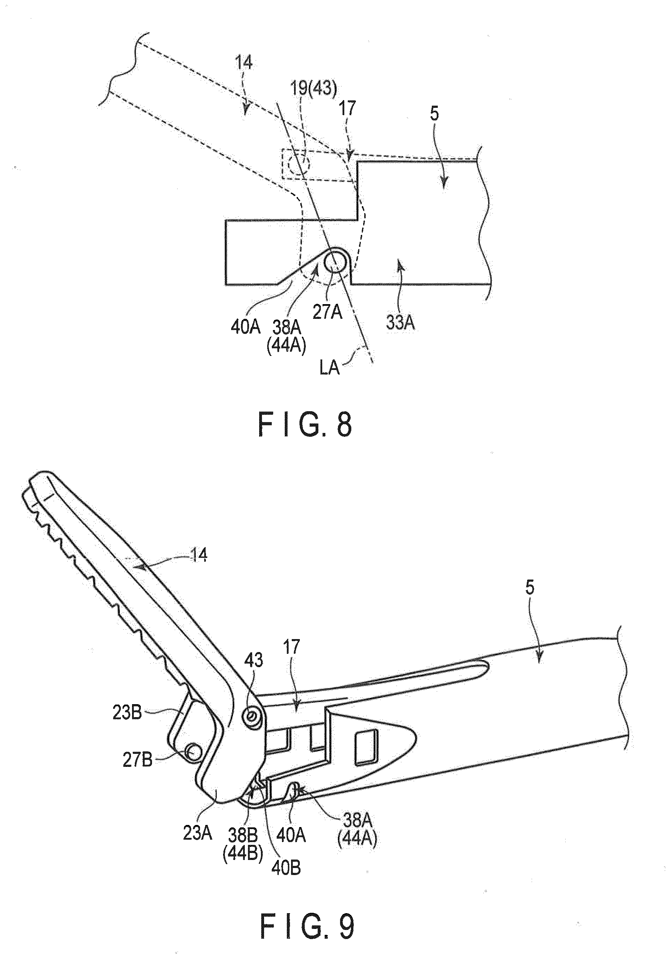

Next, a second embodiment of the present invention will be described with reference to FIGS. 8 and 9. The second embodiment is prepared by modifying the configuration of the first embodiment as follows. The same parts as those of the first embodiment are denoted by the same reference numerals, and descriptions thereof are omitted.

FIG. 8 is a schematic diagram showing a state in the present embodiment where a movable member 17 has moved to the most proximal side relative to a sheath 5 and a housing 4 (namely, a state where a handle 12 is the most open relative to a grip 11) as viewed from one side of the width direction. As shown in FIG. 8, in a cross section intersecting with the width direction, a straight line passing through a first connecting portion 43 and a second connecting portion 44A (44B) is defined as a straight line LA (LB). In this modification, a hole 38A (38B) opens in the direction intersects with the width direction of a second grasping piece 14 and intersecting with (or substantially perpendicular to) the straight line LA (LB) in a state where the second grasping piece 14 is the most open relative to the first grasping piece 13. In addition, in the holes 38A and 38B, the cross-sectional area that intersects with (or substantially perpendicular to) the direction in which the hole 38A, 38B opens increases as it is advanced toward an opening 40A, 40B.

Also in the present embodiment, the hole 38A, 38B provided in the sheath 5 is opened in the direction intersecting with the protruding direction of the protrusion 27A, 27B provided in the second grasping piece 14. Therefore, also in this modification, the second grasping piece 14 can be easily attached to the sheath 5 as in the first embodiment.

When the grasping treatment instrument 1 is used, an excessive force may act on the second grasping piece 14 in a direction in which the second grasping piece 14 opens relative to the first grasping piece 13. At this time, the second grasping piece 14 moves rotationally relative to the sheath 5 with the second connecting portion 44A, 44B as a fulcrum and opens relative to the first grasping piece 13. By causing the second grasping piece 14 to move rotationally relative to the sheath 5 with the second connecting portion 44A, 44B as a fulcrum, the movable member 17 moves to the proximal side relative to the sheath 5. Then, in a state where the movable member 17 is positioned on the most proximal side relative to the sheath 5 (a state where the handle 12 is the most open relative to the grip 11), the movement of the movable member 17 relative to the sheath 5 is stopped. At this time, the second grasping piece 14 is in a state of being the most open relative to the first grasping piece 13. That is, the angle between the second grasping piece 14 and the first grasping piece 13 becomes the largest angle (predetermined angle).

In a state where the second grasping piece 14 is the most open relative to the first grasping piece 13, the opening movement of the handle 12 relative to the grip 11 is restricted, so that the moving of the movable member 17 toward the proximal side relative to the sheath 5 is restricted. Therefore, in the state where the second grasping piece 14 is the most open relative to the first grasping piece 13, when a force acts on the second grasping piece 14 in a direction in which the second grasping piece 14 opens, a moment force causing the second grasping piece 14 to move rotationally in a direction it opens, relative to the sheath 5, acts using the first connecting portion 43 as a fulcrum. Then, a force causing the protrusion 27A, 27B to move along the axial direction centering on the first connecting portion 43 acts on the protrusion 27A, 27B. That is, a force to move the protrusion 27A, 27B in a direction intersecting with (or substantially perpendicular to) the straight line LA, LB that passes through the first connecting portion 43 and the second connecting portion 44A, 44B (protrusion 27A, 27B) acts on the protrusion 27A, 27B.

In the present embodiment, in a cross section that intersects with the width direction, in a state where the second grasping piece 14 is the most open relative to the first grasping piece 13, the hole 38A, 38B is opened in a direction intersecting with the straight line LA (LB) that passes through the first connecting portion 43 and the second connecting portion 44A, 44B. That is, the hole 38A, 38B is opened in a direction in which the protrusion 27A, 27B moves axially centering on the first connecting portion 43. Therefore, the protrusion 27A, 27B moves centering on the first connecting portion 43 from the state where the second grasping piece 14 is the most open relative to the first grasping piece 13, whereby the protrusion 27A, 27B moves in the hole 38A, 38B toward the opening 40A, 40B. The protrusion 27A, 27B to move in the hole 38A, 38B toward the opening 40A, 40B, so that the second grasping piece 14 moves rotationally relative to the sheath 5 using the first connecting portion 43 as a fulcrum. Then, as shown in FIG. 9, the protrusion 27A, 27B passes through the opening 40A, 40B and comes out to the outside of the hole 38A, 38B. When the protrusion 27A and 27B comes out from the hole 38A, 38B, the second grasping piece 14 and the sheath 5 disengage from each other at the second connecting portion 44A, 44B. The engagement between the second grasping piece 14 and the sheath 5 is released at the second connecting portions 44A and 44B, whereby the second grasping piece 14 can move rotationally relative to the movable member 17 using the first connecting portion 43 as a fulcrum.

The cross-sectional area of the hole 38A, 38B intersecting with (or perpendicular to) the direction in which the hole 38A, 38B opens increases as it is advanced toward the opening 40A, 40B. Therefore, when an excessive force acts on the second grasping piece 14 in a direction in which the second grasping piece 14 opens relative to the first grasping piece 13, the protrusion 27A, 27B can easily come out from the opening 40A, 40B to the outside of the hole 38A, 38B.

As described above, in the present embodiment, when an excessive force acts on the second grasping piece 14 in a direction in which the second grasping piece 14 opens relative to the first grasping piece 13, the engagement (connection) between the second grasping piece 14 and the sheath 5 is released at the second connecting portion 44A, 44B, whereby the second grasping piece 14 can move rotationally relative to the movable member 17 using the first connecting portion 43 as a fulcrum. The second grasping piece 14 moves rotationally relative to the movable member 17 using the first connecting portion 43 as a fulcrum, whereby the excessive force acting on the second grasping piece 14 is prevented from being transmitted to the internal mechanism, etc. of the housing 4 (e.g., a mechanism that transmits an operational force from the handle 12 to the second grasping piece 14) via the first connecting portion 43 and the movable member 17. The excessive force acting on the second grasping piece 14 is prevented from being transmitted to the internal mechanism, etc. of the housing 4 via the first connecting portion 43 and the movable member 17, whereby breakage is effectively prevented from occurring in the first connecting portion 43, the second connecting portion 44A, 44B, and the inside of the housing 4, etc.

### (First Modification of Second Embodiment)

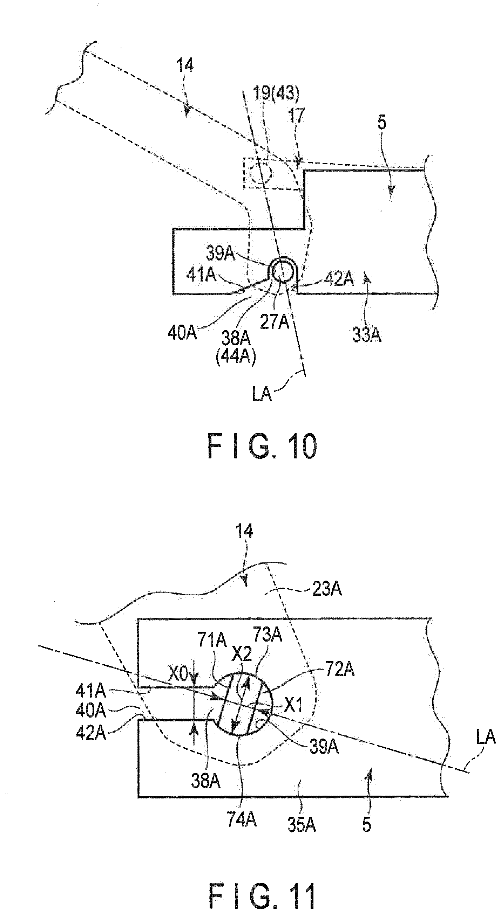

FIG. 10 is a schematic diagram showing a state as viewed from one side of the width direction in a first modification of the second embodiment where the movable member 17 has moved to the most proximal side relative to the sheath 5 and the housing 4 (namely, a state where the handle 12 is the most open relative to the grip 11). As shown in FIG. 10, the hole 38A (38B) has a circular arc portion 39A (39B), a flat surface portion 41A, 41B (a first flat surface), and a flat surface portion 42A, 42B (a second flat surface). In a cross section intersecting with (or substantially perpendicular to) the width direction, the flat surface portion 41A, 41B extends from the end portion (one end) on the proximal side of the circular arc portion 39A (39B) toward the side where the second grasping piece 14 closes relative to the first grasping piece 13. The flat surface portion 42A (42B) extends from the end portion (the other end) on the distal side of the arc portion 39A (39B) toward a direction intersecting with (or substantially perpendicular to) a straight line LA (LB). An opening 40A (40B) is formed by a space between the flat surface portion 41A (41B) and the flat surface portion 42A (42B). The distance between the flat surface portion 41A (41B) and the flat surface portion 42A (42B) increases as it is advanced from the circular arc portion 39A (39B) toward the opening 40A (40B).

As described above, the protrusion 27A, 27B moves toward the opening 40A, 40B of the hole 38A, 38B as the second grasping piece 14 opens relative to the first grasping piece 13. In this modification, the protrusion 27A, 27B is in contact with the circular arc portion 39A, 39B at the moment when or until the time immediately before the second grasping piece 14 is the most open relative to the first grasping piece 13. Then, in a state where the second grasping piece 14 is the most open relative to the first grasping piece 13, the protrusion 27A, 27B is placed at a position not in contact with the circular arc portion 39A, 39B (See FIG. 10). Therefore, in this modification, only in the state where the second grasping piece 14 is the most open relative to the first grasping piece 13, the protrusion 27A, 27B moves in the hole 38A, 38B toward the opening 40A, 40B, and the engagement (connection) between the second grasping piece 14 and the sheath 5 can be released at the second connecting portion 44A, 44B. Therefore, when an excessive force acts on the second grasping piece 14 in a direction in which the second grasping piece 14 opens relative to the first grasping piece 13, breakage is effectively prevented from occurring in the first connecting portion 43, the second connecting portion 44A, 44B, and the inside of the housing 4, etc.

### (Second Modification of Second Embodiment)

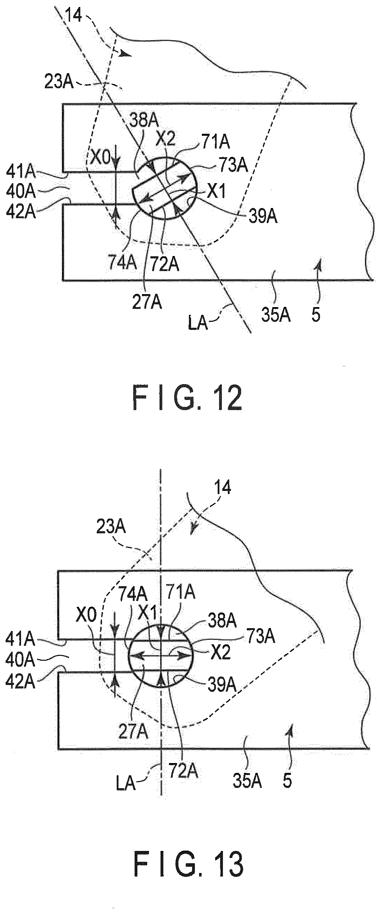

FIG. 11 is a schematic diagram of the second connecting portion 44A (44B) in a state where a second grasping piece 14 is closed to some extent relative to the first grasping piece 13 as viewed from one side of the width direction in a second modification of the second embodiment. FIG. 12 is a schematic diagram of the second connecting portion 44A (44B) as viewed from one side in the width direction in a state where the second grasping piece 14 is opened relative to the first grasping piece 13 to some extent from the state shown in FIG. 11. FIG. 13 is a schematic diagram of the second connecting portion 44A (44B) as viewed from one side in the width direction in a state where the second grasping piece 14 is the most open relative to the first grasping piece 13 (a state where the second grasping piece 14 further opened from the state shown in FIG. 12). As shown in FIGS. 11 to 13, also in this modification, similarly to the first modification of the second embodiment, the hole 38A, 38B of the sheath 5 includes a circular arc portion 39A, 39B, a flat surface portion 41A, 41B, and a flat surface portion 42A, 42B. Here, in the hole 38A, 38B, the size (the width of an opening) between the flat surface portion 41A, 41B and the flat surface portion 42A, 42B in the direction substantially perpendicular to the flat surface portion 41A, 41B and the flat surface portion 42A, 42B (in the width direction of the opening) is defined as X0.

On the protrusion 27A (27B) of the second grasping piece 14, a flat surface portion 71A (71B) and a flat surface portion 72A (72B) extend along the protruding direction of the protrusion 27A (27B). The flat surface portion 71A (71B) and the flat surface portion 72A (72B) extend substantially in parallel with each other. In a cross section intersecting with (or substantially perpendicular to) the width direction (the protruding direction of the protrusion 27A (27B)), a straight line is formed by the flat surface portions 71A (71B) and 72A (72B). On the outer peripheral surface of the protrusion 27A (27B), a curved surface portion 73A (73B) and a curved surface portion 74A (74B) are formed between the flat surface portion 71A (71B) and the flat surface portion 72A (72B). In a cross section intersecting with (or substantially perpendicular to) the width direction, a circular arc-shaped curve is formed by the curved surface portion 73A (73B) and the curved surface portion 74A (74B).

Here, in a cross section intersecting with (or substantially perpendicular to) the width direction, a direction substantially perpendicular to the straight line formed by the flat surface portions 71A (71B) and 72A (72B) is defined as a "first direction", and a direction intersecting with (or substantially perpendicular to) the first direction is defined as a "second direction". Furthermore, in the cross section intersecting with (or substantially perpendicular to) the width direction, a size of the protrusion 27A (27B) in the first direction (namely, the distance between the flat surface portion 71A (72B) and the flat surface portion 72A (72B)) is defined as X1 (first size), and a size of the protrusion 27A (27B) in the second direction is defined as X2 (second size). In this modification, the flat surface portions 71A (71B) and 72 A (72 B) are provided on the protrusion 27A (27B) so that X1 ≤ X0 < X2. That is, in the cross section intersecting with (or substantially perpendicular to) the width direction, the size (width) of the protrusion 27A (27B) is set so that only the size (X1) in the first direction is smaller than the width (X0) of the opening of the hole 38A (38B). Furthermore, as shown in FIG. 13, in a state where the second grasping piece 14 is the most open relative to the first grasping piece 13, the flat surface portions 71A (71B) and 72A (72B) are provided on the protrusion 27A (27B) so that the first direction of the protrusion 27A (27B) is substantially in parallel with the width direction of the opening of the hole 38A. That is, in a state where the second grasping piece 14 is the most open relative to the first grasping piece 13, the size of the protrusion 27A (27B) in the width direction of the opening of the hole 38A (38B) becomes X1. Therefore, only in the state where the second grasping piece 14 is the most open relative to the first grasping piece 13, the size of the protrusion 27A (27B) in the width direction of the opening of the hole 38A (38B) is smaller than the width (X0) of the opening of the hole 38A (38B).

In a state where the second grasping piece 14 is closed to some extent relative to the first grasping piece 13, the curved surface portions 73A, 73B and the curved surface portions 74A, 74B of the protrusions 27A, 27B contact the circular arc portion 39A, 39B of the hole 38A, 38B at the second connecting portion 44A, 44B (see FIG. 11). When the second grasping piece 14 opens relative to the first grasping piece 13 from the state shown in FIG. 11, the protrusion 27A, 27B of the second grasping piece 14 rotates relative to the sheath 5 in the hole 38A, 38B of the sheath 5 (see FIG. 12). As described above, in this modification, only in the state where the second grasping piece 14 is the most open relative to the first grasping piece 13, the size of the protrusion 27A, 27B in the width direction of the opening of the hole 38A, 38B is smaller than the width (X0) of the opening of the hole 38A, 38B. Therefore, only in the state where the second grasping piece 14 is the most open relative to the first grasping piece 13, the protrusion 27A, 27B can move toward the opening of the hole 38A, 38B. That is, only in the state where the second grasping piece 14 is the most open relative to the first grasping piece 13, the engagement (connection) between the second grasping piece 14 and the sheath 5 can be released at the second connecting portions 44A and 44B.

Also in this modification, when an excessive force acts on the second grasping piece 14 in a direction in which the second grasping piece 14 opens relative to the first grasping piece 13, the engagement (connection) between the second grasping piece 14 and the sheath 5 is released at the second connecting portion 44A, 44B, whereby breakage is effectively prevented from occurring in the first connecting portion 43, the second connecting portion 44A, 44B, and the inside of the housing 4, etc. Furthermore, in this modification, only in the state where the second grasping piece 14 is the most open relative to the first grasping piece 13, the engagement (connection) between the second grasping piece 14 and the sheath 5 can be released at the second connecting portion 44A, 44B. Therefore, in this modification, in a state other than the case where the second grasping piece 14 is the most open relative to the first grasping piece 13, release of the engagement (connection) between the second grasping piece 14 and the sheath 5 is effectively prevented.

### (Third Embodiment)

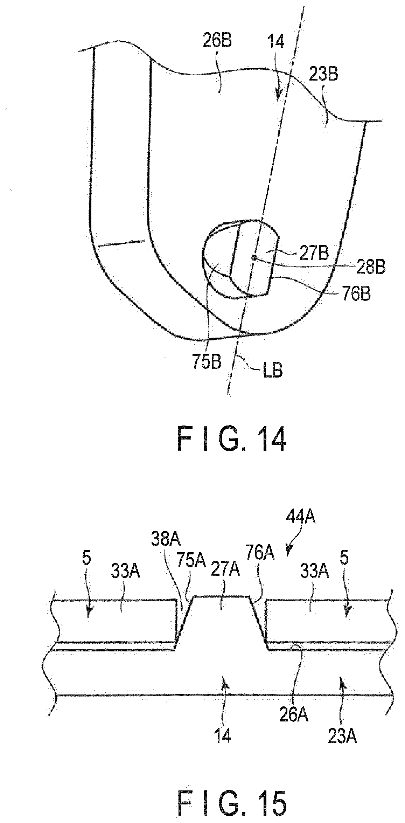

Next, a third embodiment of the present invention will be described with reference to FIGS. 14 to 16. The third embodiment is prepared by modifying the configuration of the first embodiment as follows. The same parts as those of the first embodiment are denoted by the same reference numerals, and descriptions thereof are omitted.

FIG. 14 is a diagram showing a protrusion 27B (27A) of a second grasping piece 14 in the third embodiment. As shown in FIG. 14, a protrusion 27B (27A) includes an inclined surface 75B (75A) and an inclined surface 76B (76A) directed toward a center axis 28B (28A) of the protrusion 27B (27A), as it is advanced from an opposed surface 26B (26A) toward the protruding direction of the protrusion 27B (27A). The inclined surface 75B (75A) and the inclined surface 76B (76A) are provided on the opposite side to each other relative to the center axis 28B (28A). Furthermore, the inclined surface 75B (75A) and the inclined surface 76B (76A) are provided to be substantially in parallel with a straight line LB (LA) passing through a first connecting portion 43 and a second connecting portion 44B (44A) in a cross section intersecting with (or substantially perpendicular to) the width direction of the second grasping piece 14. That is, the protrusion 27B (27A) includes inclined surfaces 75B (75A) and 76B (76A) facing a direction intersecting with (or substantially perpendicular to) the straight line LB (LA).

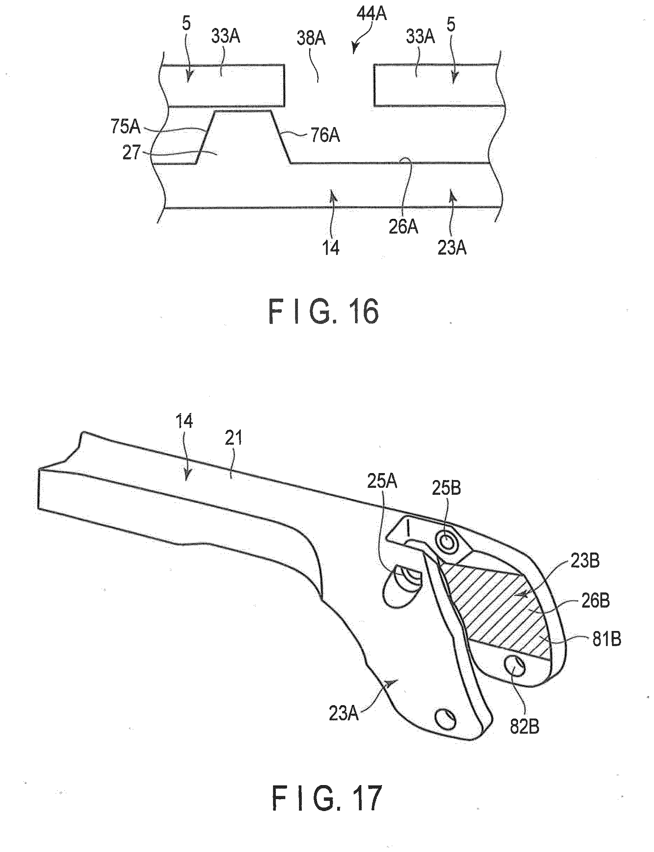

FIG. 15 is a diagram showing the second connecting portion 44A (44B) in a cross section that intersects with the opening/closing direction of the second grasping piece 14. FIG. 16 is a diagram showing a cross section that intersects with the opening/closing direction of the second grasping piece 14 in a state where the second grasping piece 14 and the sheath 5 are disengaged. As shown in FIG. 15, in a state where the protrusion 27A (27B) is engaged with a hole 38A (38B) at the second connecting portion 44A (44B), the inclined surface 75B (75A) and the inclined surface 76B (76A) respectively contact the edge of the hole 38A (38B). When the second grasping piece 14 opens relative to the first grasping piece 13, a force to move the protrusion 27A (27B) in a direction intersecting with (or substantially perpendicular to) the straight line LA (LB) acts on the protrusion 27A (27B). In the present embodiment, a force to move the protrusion 27A (27B), in a direction in which the inclined surface 75A (75B) faces, acts on the protrusion 27A (27B).

In the present embodiment, when an excessive force acts on the second grasping piece 14 in a direction in which the second grasping piece 14 opens relative to the first grasping piece 13, an excessive force causing the protrusion 27A (27B) to move the protrusion 27A (27B), in a direction in which the inclined surface 75A (75B) faces, acts on the protrusion 27A (27B) at a contact portion between the inclined surface 75A (75B) and the hole 38A (38B). With this, at the second connecting portion 44A (44B), the protrusion 27A (27B) moves outward relative to the sheath 5 in the width direction of the second grasping piece 14 along the inclined surface 75A (75B). That is, in the inclined surface 75A (75B), the contact portion between the inclined surface 75A (75B) and the edge of the hole 38A (38B) moves along the inclined surface 75A (75B) toward the opposite side of the protruding direction of the protrusion 27A (27B). Therefore, a projecting piece 23A (23B) of the second grasping piece 14 moves outward relative to the sheath 5 in the width direction. Then, the protrusion 27A (27B) comes out to the outside of the hole 38A (38B) (see FIG. 16). As the protrusion 27A (27B) comes out to the outside of the hole 38A (38B), the engagement (connection) between the second grasping piece 14 and the sheath 5 is released at the second connecting portions 44A and 44B.

Therefore, also in the present embodiment, when an excessive force acts on the second grasping piece 14 in the direction in which the second grasping piece 14 opens relative to the first grasping piece 13, the engagement (connection) between the second grasping piece 14 and the sheath 5 is released at the second connecting portion 44A, 44B. The engagement (connection) between the second grasping piece 14 and the sheath 5 is released, whereby breakage is effectively prevented from occurring in the first connecting portion 43, the second connecting portion 44A, 44B, and the inside of the housing 4, etc., similar to the second embodiment.

Furthermore, in the present embodiment, in a state where the second grasping piece 14 is attached to the sheath 5, the same effect can be obtained even when an excessive force acts on the second grasping piece 14, in a direction in which the second grasping piece 14 closes relative to the first grasping piece 13. In this case, an excessive force to move the protrusion 27A (27B), in a direction in which the inclined surface 76A (76B) faces, acts on the protrusion 27A (27B) at the contact portion between the inclined surface 76A (76B) and the hole 38A (38B), and the engagement (connection) between the second grasping piece 14 and the sheath 5 is released at the second connecting portion 44A, 44B. The engagement (connection) between the second grasping piece 14 and the sheath 5 is released, whereby breakage is effectively prevented from occurring in the first connecting portion 43, the second connecting portion 44A, 44B, and the inside of the housing 4, etc.

Using the grasping treatment instrument 1 according to the present embodiment, etc., a treatment target such as a living tissue is grasped between the first grasping piece 13 and the second grasping piece 14, and treatment energy is applied to the grasped treatment target, whereby the treatment target may be sealed (and incised). In one embodiment, a high frequency current is used as the treatment energy. In this case, an unillustrated electrode is provided on each of the first grasping piece 13 and the second grasping piece 14. When electric energy is supplied to these electrodes from an unillustrated power supply unit, a high-frequency current flows between the electrodes through the treatment target.

In another embodiment, ultrasonic vibration is used as a treatment energy. In this case, an unillustrated ultrasonic transducer is provided inside the housing 4, and an unillustrated probe extending toward the distal side through the inside of the sheath 5 is connected to the ultrasonic transducer. Then, the first grasping piece 13 is formed by the projecting portion of the probe from the sheath 5. Electric energy is supplied from the power supply unit to the ultrasonic transducer, and ultrasonic vibration is generated in the ultrasonic transducer. The generated ultrasonic vibration is transmitted (applied) to a treatment target via the probe (the first grasping piece 13) .

In yet another embodiment, heat generated by an unillustrated heat generator provided on the end effector is used as a treatment energy. In this case, electric energy is supplied from a power supply unit 46 of an energy controller 3 to the heat generator, and the heat generated by the heating element is applied to the treatment target.

In addition, a plurality of treatment energies among a high-frequency current, ultrasonic vibration, heat generated by a heat generator, etc., may be simultaneously applied to a treatment target to be grasped.

In the above-described embodiments, etc., the grasping treatment instrument (1) includes a sheath (5) extending along the longitudinal axis C; a first grasping piece (13) provided at a distal end portion of the sheath (5); a second grasping piece (14) attached to the distal end portion of the sheath (5) so as to be rotationally movable relative to the sheath (5); a protrusion (27A, 27B) provided on the second grasping piece (14) and protruding in a direction intersecting with the longitudinal axis C and intersecting with the opening/closing direction of the second grasping piece (14) relative to the first grasping piece (13); and a recess (38A, 38B) that engages with the protrusion (27A, 27B) at the distal end portion of the sheath and has a recessed shape that opens in a direction intersecting with the direction in which the protrusion (27A, 27B) protrudes.

### (Reference Embodiment)

Next, referring to FIG. 17, a reference embodiment of the present invention will be described. The same parts as those of the embodiments, etc. of the present invention are denoted by the same reference numerals, and the descriptions thereof will be omitted. Using the grasping treatment instrument 1 according to the present embodiment, etc., a treatment target such as a living tissue is grasped between the first grasping piece 13 and the second grasping piece 14, and the high-frequency current is made to flow into the grasped treatment target, whereby the treatment target may be sealed (and incised). In this case, each of the sheath 5 and the movable member 17 includes a conductive part, and the first grasping piece 13 includes an electrode (first electrode) formed of a conductive material. In the second grasping piece 13, a support member 21 is formed of a conductive material, and the clamp part 22 includes an electrode (second electrode) formed of a conductive material.

Furthermore, the grasping treatment instrument 1 is connected to an unillustrated power supply unit via an unillustrated cable, etc. Then, the electric energy (high-frequency energy) output from the power supply unit is supplied to the electrode of the first grasping piece 13. The electric energy output from the power supply unit is supplied to the second grasping piece 14 through the movable member 17 and is supplied to the electrode of the clamp part 22 through a support member 21 in the second grasping piece 14. Electric energy is supplied from an energy output source to the first grasping piece 13 and the clamp part 22, in a state where a treatment target is grasped between the grasping pieces 13 and 14, whereby a high-frequency current flows between the first grasping piece 13 and the clamp part 22 through the grasped treatment target.

FIG. 17 is a diagram showing the support member 21 of the second grasping piece 14 in this reference example. As shown in FIG. 17, a coating portion 81B (the hatched area in FIG. 17) is provided on an opposed surface 26B of a projecting piece 23B. The coating portion 81B is subjected to insulating coating processing, and the coating portion 81B has electrically insulating properties. As can be seen from FIG. 17, the coating processing is not performed on a peripheral portion of a hole 25B (a connecting portion at which it is connected to the movable member 17) and a peripheral portion of a hole 82B (the connecting portion at which it is connected to the sheath 5) of an opposed surface 26B. Therefore, the electric energy output from the power supply unit is effectively supplied to the support member 21 via the sheath 5 and the movable member 17. Similar to the opposed surface 26B, an insulating coating portion 81A is also provided to the opposed surface 26A, and the coating treatment described above is performed.

In this reference embodiment, when electric energy output from the power supply unit is supplied to the first grasping piece 13, the support member 21 and the clamp part 22, even when a conductive substance (physiological saline, blood, etc.) is present between the first grasping piece 13 and the opposed surface 26A, 26B of the projecting piece 23A, 23B, a high-frequency current is prevented from flowing between the first grasping piece 13 and the projecting piece 23A, 23B through the conductive substance, because the electrically insulating coating portion 81A, 81B is provided on the opposed surface 26A, 26B. By preventing a high-frequency current from flowing between the first grasping piece 13 and the projecting pieces 23A, 23B, it is possible to pass a desired high-frequency current through a treatment target grasped between the first grasping piece 13 and the second grasping piece 14. A desired high-frequency current flows through the treatment target grasped between the first grasping piece 13 and the second grasping piece 14, whereby a desired treatment (sealing or incision) performance for the treatment target can be obtained.

Although the embodiments, etc. of the present invention have been described above, the present invention is not limited to the above-described embodiments, etc., and, needless to say, various modifications can be made without departing from the spirit of the invention.

## Claims

1. A grasping treatment instrument comprising:
a sheath having a longitudinal axis and extending along the longitudinal axis;
a first grasping piece provided at a distal end portion of the sheath:
a second grasping piece attached to the distal end portion of the sheath so as to be rotationally movable relative to the sheath, the second grasping piece moving rotationally relative to the sheath, thereby opening and closing relative to the first grasping piece to grasp a living tissue between the second grasping piece and the first grasping piece;
a protrusion provided on the second grasping piece and protruding in a direction intersecting with the longitudinal axis and intersecting with a direction in which the second grasping piece opens and closes relative to the first grasping piece; and
a recess that engages with the protrusion at the distal end portion of the sheath and has a recessed shape that opens in a direction intersecting with the direction to which the protrusion protrudes.

2. The grasping treatment instrument according to claim 1, further comprising:
a movable member that extends along the longitudinal axis of the sheath, is connected to the second grasping piece at a region different from a region of the protrusion, and is configured to move relative to the sheath, thereby rotationally moving the second grasping piece relative to the sheath,
wherein the second grasping piece is able to open up to a predetermined angle relative to the first grasping piece, and
wherein the recessed shape of the recess opens in a direction intersecting with a straight line passing through the recess and a connecting portion between the movable member and the second grasping piece, in a state where the second grasping piece opens up to the predetermined angle relative to the first grasping piece.

3. The grasping treatment instrument according to claim 2, wherein the recess includes, in a cross section intersecting with the direction to which the protrusion protrudes, a circular arc-shaped curved surface, a first flat surface that extends from one end of the curved surface toward an opening of the recess, and a second flat surface that extends from another end of the curved surface toward the opening and that is set apart from the first flat surface as being advanced toward the opening.

4. The grasping treatment instrument according to claim 2, wherein the protrusion has a first size that is equal to or smaller than a width of the opening of the recess, in a first direction that intersects with the direction to which the protrusion protrudes, and a second size that is larger than the width of the opening, in a second direction that intersects with the direction to which the protrusion protrudes and intersects with the first direction, and
wherein in a state where the second grasping piece opens up to the predetermined angle relative to the first grasping piece, the first direction is in parallel with a width direction of the opening.

5. The grasping treatment instrument according to claim 1, wherein the protrusion has a circular arc-shaped curved surface in a cross section intersecting with the direction to which the protrusion protrudes.

6. The grasping treatment instrument according to claim 5, wherein in the cross section intersecting with the direction to which the protrusion protrudes, the recess has a circular arc-shaped curved surface, and
wherein in a state where the second grasping piece is moving rotationally relative to the sheath, and the curved surface of the protrusion is in contact with the curved surface of the recess.

7. The grasping treatment instrument according to claim 1, wherein the recess has a circular arc-shaped curved surface in a cross section intersecting with the direction to which the protrusion protrudes.

8. The grasping treatment instrument according to claim 1, wherein the protrusion moves toward an opening of the recess having the recessed shape as the second grasping piece opens relative to the first grasping piece.

9. The grasping treatment instrument according to claim 1, further comprising:
a movable member that extends along the longitudinal axis of the sheath, is connected to the second grasping piece at a region different from a region of the protrusion, and is configured to move relative to the sheath, thereby rotationally moving the second grasping piece relative to the sheath,
wherein the recess is positioned, relative to the longitudinal axis, on an opposite side of a connecting portion at which the second grasping piece is connected to the movable member.

10. The grasping treatment instrument according to claim 1, further comprising:
a movable member that extends along the longitudinal axis of the sheath, is connected to the second grasping piece at a region different from a region of the protrusion, and is configured to move relative to the sheath, thereby rotationally moving the second grasping piece relative to the sheath,
wherein the protrusion has a center axis along the direction to which the protrusion protrudes, and
wherein the protrusion includes an inclined surface that is directed toward the center axis of the protrusion as the inclined surface is advanced toward the direction to which the protrusion protrudes, and that faces a direction intersecting with a straight line passing through the protrusion and a connecting portion between the movable member and the second grasping piece.
